# EUROPEAN PATENT APPLICATION

(11) **EP 2 055 297 A1**
(43) Date of publication of application: **06.05.2009**
(21) Application number: 07120015.8
(22) Date of filing: 05.11.2007
(51) Int. Cl.: A61K 8/55, A61K 31/663, A61Q 5/10

(54) **Therapy option for recoloration of hair via bisphosphonates by physiological repigmentation with age-related and/or premature "grayed" patients**

(71) Applicant: Merz Pharma GmbH & Co. KGaA, 60318 Frankfurt Main (DE)
(72) Inventor: Köhler, Holger, 55218, Ingelheim (DE); Pooth, Rainer, 65812, Bad Soden (DE)
(74) Representative: Michalski Hüttermann & Partner Patentanwälte

(57) **Abstract**

The present invention relates to the use of a bisphosphonate derivative according to general Formula I 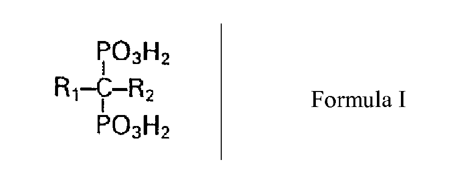 and/or a pharmaceutically acceptable salt thereof,
wherein R₁ and/or R₂ are independently selected out of a group comprising hydrogen, hydroxyl, halogen, pseudohalogen, formyl, carboxy- and/or carbonyl derivatives, alkyl, long-chain alkyl, alkoxy, long-chain alkoxy, cycloalkyl, halogenalkyl, aryl, arylene, halogenaryl, heteroaryl, heteroarylene, heterocycloalkylene, heterocycloalkyl, halogenheteroaryl, alkenyl, halogenalkenyl, alkinyl, halogenalkinyl, keto, ketoaryl, halogenketoaryl, ketoheteroaryl, ketoalkyl, halogenketoalkyl, ketoalkenyl, halogenketoalkenyl, phosphoalkyl, phosphonate, phosphate, phosphine, phosphine oxide, phosphoryl, phosphoaryl, sulphonyl, sulphoalkyl, sulphoarenyl, sulphonate, sulphate, sulphone, amine, polyether, silylalkyl, silylalkyloxy, and wherein at appropriate residues one or more non-adjacent CH₂-groups may be replaced independently from each other with -O-, -S-, -NH-, -NR°-, -SiR°R°°-, -CO-, -COO-, -OCO-, -OCO-O-, -SO₂-, -S-CO-, -CO-S-, -CY¹=CY² or -C=C- in that way that O and/or S-atoms are not connected directly with each other, likewise replaced optionally with aryl- or heteroaryl which preferably contain 1 to 30 C-atoms (terminal CH₃-groups are meant as CH₂-groups in terms of CH₂-H), wherein R°, R°°, Y¹ and Y² are independently selected out of a group comprising alkyl and aryl,
AH:SH
in the manufacture of a pharmaceutical for the treatment of age-related and/or premature graying of hair.

## Description

The present invention relates to a therapy option for recoloration of hair via bisphosponates by physiological repigmentation with age-related and/or premature "grayed" patients.

The appearance of hair plays an important role in people's overall physical appearance and self-perception. With today's increasing life expectation, the desire to look youthful plays a bigger role than ever. The hair care industry has become aware of this and also more capable to deliver active products that are directed toward meeting this consumer demand. Topical anti-aging compounds for hair include humefactants, hair conditioners, photoprotectors, and antioxidants (Trüeb, 2005).

Human hair color is affected by various factors. Therein, the content of the pigments of the hair shaft and physical effects are essential. Physical effects comprise the reflection and absorption of light which are regulated by the thickness of the hair, the shape of the shaft and the ultrastructure. The impression of white hair arises in particular in the absence of the pigment melanin by the reflection of light. The impression of black hair in turn arises by the absorption of light.

Hair color has a spectrum from gray, yellow, red, brown to black. Three classes of the pigment melanin are known: eumelanin, pheomelanin and neuromelanin. The black-brown eumelanins, the red-yellow pheomelanins and the trichochromes which belong to the pheomelanins and primarily may be found in human red hair are essential for the development of hair color (Agrup et al., 1978, Slominski et al., 2005).

The phylogenetically ancient biochemical pathway underlying the hair color is called melanogenesis and results in the production of melanin pigments in neural crest-derived melanocytes. While melanin from epidermal melanocytes clearly protects human skin by screening harmful ultraviolet radiation, the biological value of hair pigmentation is less clear. One potential benefit of pigmented scalp hair in humans may be the rapid excretion of heavy metals, chemicals, toxins from the body by their selective binding to melanin. It is suggested that the appearance of gray and white hair is caused by an age-related, genetically regulated exhaustion of the pigmentary potential of each individual hair follicle. Melanocyte aging may be associated with reactive oxygen species-mediated damage to nuclear and mitochondrial DNA with resultant accumulation of mutations with age, in addition to dysregulation of antioxidant mechanisms or "gray hair" derives in large part from the admixture of pigmented and white hair. It is important to note that individual hair follicles can indeed exhibit pigment dilution or true grayness. This dilution is due to a reduction in tyrosinase activity of hair bulbar melanocytes, sub-optimal melanocyte-cortical keratinocyte interactions, and defective migration of melanocytes from a reservoir in the upper outer root sheath to the pigment-permitting microenvironment close to the dermal papilla of the hair bulb. One of the surprising results of pigment loss is the alteration in keratinocyte proliferation and differentiation, providing the tantalizing suggestion that the melanocytes in the hair follicle contribute far more that packages of melanin alone (Tobin and Paus, 2001).

To date there exists no therapy for repigmentation of "grayed" hair. In the absence of another way to reverse hair graying, hair colorants are the mainstays of recovering lost hair color (Trüeb, 2005). However, hair colorants exhibit some disadvantages. The effects of hair colorants or tinge agents are only temporary and achieve rarely the same visual effect than the natural hair color. Frequently the hair colorants impair the hair quality due to the admixture of oxidizing agents.

The problem underlying the present invention is to provide a therapy option for recoloration of hair by physiological repigmentation with age-related and/or premature "grayed" patients.

Subjects are classified as having premature graying when the subjects' hair had turned more than 50 % gray before 40 years of age (Rosen et al., 1994).

The solution solving the problem underlying the present invention is provided by the present invention. Accordingly, a use of a bisphosphonate derivative is provided according to general Formula I 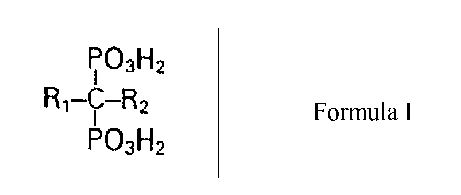 and/or a pharmaceutically acceptable salt thereof,
wherein R₁ and/or R₂ are independently selected out of a group comprising hydrogen, hydroxyl, halogen, pseudohalogen, formyl, carboxy- and/or carbonyl derivatives, alkyl, long-chain alkyl, alkoxy, long-chain alkoxy, cycloalkyl, halogenalkyl, aryl, arylene, halogenaryl, heteroaryl, heteroarylene, heterocycloalkylene, heterocycloalkyl, halogenheteroaryl, alkenyl, halogenalkenyl, alkinyl, halogenalkinyl, keto, ketoaryl, halogenketoaryl, ketoheteroaryl, ketoalkyl, halogenketoalkyl, ketoalkenyl, halogenketoalkenyl, phosphoalkyl, phosphonate, phosphate, phosphine, phosphine oxide, phosphoryl, phosphoaryl, sulphonyl, sulphoalkyl, sulphoarenyl, sulphonate, sulphate, sulphone, amine, polyether, silylalkyl, silylalkyloxy, and wherein at appropriate residues one or more non-adjacent CH₂-groups may be replaced independently from each other with -O-, -S-, -NH-, -NR°-, -SiR°R°°-, -CO-, -COO-, -OCO-, -OCO-O-, -SO₂-, -S-CO-, -CO-S-, -CY¹=CY² or -C=C- in that way that O and/or S-atoms are not connected directly with each other, likewise replaced optionally with aryl- or heteroaryl which preferably contain 1 to 30 C-atoms (terminal CH₃-groups are meant as CH₂-groups in terms of CH₂-H), wherein R°, R°°, Y¹ and Y² are independently selected out of a group comprising alkyl and aryl, in the manufacture of a pharmaceutical for the treatment of age-related and/or premature graying of hair. Likewise, the present invention is also directed to a pharmaceutical composition comprising a compound according to Formular I for the treatment of age-related and/or premature graying of hair.

Furthermore, the present invention relates to a process, comprising: administering a composition comprising a bisphosphonate derivative according to general Formula I 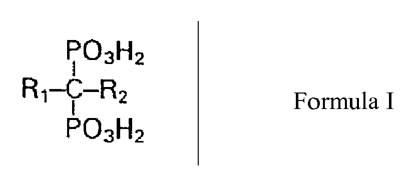 and/or a pharmaceutically acceptable salt thereof,

wherein R₁ and/or R₂ are independently selected out of a group comprising hydrogen, hydroxyl, halogen, pseudohalogen, formyl, carboxy- and/or carbonyl derivatives, alkyl, long-chain alkyl, alkoxy, long-chain alkoxy, cycloalkyl, halogenalkyl, aryl, arylene, halogenaryl, heteroaryl, heteroarylene, heterocycloalkylene, heterocycloalkyl, halogenheteroaryl, alkenyl, halogenalkenyl, alkinyl, halogenalkinyl, keto, ketoaryl, halogenketoaryl, ketoheteroaryl, ketoalkyl, halogenketoalkyl, ketoalkenyl, halogenketoalkenyl, phosphoalkyl, phosphonate, phosphate, phosphine, phosphine oxide, phosphoryl, phosphoaryl, sulphonyl, sulphoalkyl, sulphoarenyl, sulphonate, sulphate, sulphone, amine, polyether, silylalkyl, silylalkyloxy, and wherein at appropriate residues one or more non-adjacent CH₂-groups may be replaced independently from each other with -O-, -S-, -NH-, -NR°-, -SiR°R°°-, -CO-, -COO-, -OCO-, -OCO-O-, -SO₂-, -S-CO-, -CO-S-, -CY¹=CY² or -C=C- in that way that O and/or S-atoms are not connected directly with each other, likewise replaced optionally with aryl- or heteroaryl which preferably contain 1 to 30 C-atoms (terminal CH₃-groups are meant as CH₂-groups in terms of CH₂-H), wherein R°, R°°, Y¹ and Y² are independently selected out of a group comprising alkyl and aryl,
and one or more pharmaceutically acceptable carriers
to a human in an amount effective for treating age-related and/or premature graying of hair.

Bisphosphonates (also known as diphosphonates) are synthetic compounds characterized by a P-C-P group, and are thus analogs of inorganic pyrophosphates that contain an oxygen atom instead of a carbon atom. The P-C-P structure allows a great number of possible variations, mostly by changing the two lateral chains on the carbon. Small changes in the structure of the bisphosphonates can lead to extensive alterations in their physicochemical, biological, therapeutic, and toxicological characteristics. Bisphosphonates can not be destroyed enzymatically.

Most of the bisphosphonates inhibit the precipitation of calcium phosphate even at very low concentration. Bisphosphonates also slow down the dissolution of these crystals. The fact that bisphosphonates inhibit calcium phosphate crystal dissolution *in vitro* led to the hypothesis that these compounds might also act on bone resorption *in vivo* (Fleisch, 2002).

These days bisphosphonates are already useful as a therapeutic drug or preventive drug of arthritis and rheumatism due to the increase of bone resorption in addition to (tumor) bone diseases such as osteoporosis, hypercalcaemia, and Paget's disease (EP 1 008 592 A2; WO 97/44017; WO 2007/031785). These compounds prevent bone resorption by binding spontaneously to the hydroxyapatite of the bone, so that osteoclasts, for example, are unable to cleave further hydroxyapatite crystals.

Bisphosphonates are used clinically as skeletal markers in the form of ^{99m}Tc derivatives, antiosteolytic agents in patients with increased bone destruction, and inhibitors of calcification in patients with ectopic calcification and ossification.

Bisphosphonates are now the therapy of choice in Paget's disease. The compound most often used was, and possibly still is, pamidronate, given parenterally. The bisphosphonates decrease bone turnover, often to normal values, they improve the morphology of bone, and they ameliorate bone pain. The effect often persists over a long time after discontinuation of the treatment.

Various bisphosphonates have been investigated in osteoporosis, the compounds investigated most thoroughly being alendronate and risedronate. The bisphosphonates not only inhibit bone loss, but can actually also increase bone mineral density. Furthermore, the vertebral and non-vertebral fracture incidence is decreased by about one-half. Bisphosphonates are active in, among others, postmenopausal and elderly women, in men, and in corticosteroid-treated patients.

Finally, bisphosphonates are the drugs of choice to decrease bone resorption in tumor bone disease, the most common being pamidronate and clodronate. The therapy induces a diminution of bone resorption, leading to a decrease in hypercalcemia, a decrease of new osteolytic lesions and a decrease of fractures, and leading to an amelioration of pain and an improvement of the quality of life (Fleisch, 2002).

Numerous bisphosphonates are now available for therapeutical use, however, their administration and delivery remains problematic.

A most preferred mode of drug delivery for any drug is the oral administration. However, this mode of administration of bisphosphonates is limited by the low gastrointestinal absorption and by overall low gastrointestinal tolerability of bisphoshonates. Gastrointestinal absorption of the bisphosphonates is very poor and, typically, only about 1% or less of the administered dose is absorbed into the general circulation (Jeal et al., 1997). Additionally, a significant number of women treated with oral bisphosphonates were reported to develop irritation of esophageal mucosa, esophageal reflux and esophagitis (Peter et al., 1998; Peter et al., 1998). To lessen these undesirable adverse reactions, the oral administration of bisphosphonates requires a very strict regimen which is hard to follow. A noncompliance with this regimen leads to a failure of the treatment. In view of the problems encountered with oral administration of bisphosphonates which limits their utility, it is clear that other delivery mechanisms which enhance the absorption and bioavailability of these drugs are extremely advantageous for achieving and increasing a therapeutic potential of bisphosphonates.

According to one aspect to the invention, R1 is selected from the group comprising halogen, hydrogen and hydroxy and R2 is independently selected from the group comprising halogen, aryl-S-, heteroaryl-S-, aryl-NH-, heteroaryl-NH-, preferably monocyclic, 5- or 6-membered monoaza-, diaza- or thiaza-aryl-NH-, and -alkylene-R3, whereby R3 is selected from the group comprising heteroaryl, hydrogen and amino.

According to one aspect to the invention, R1 is selected from the group comprising halogen, hydrogen and hydroxy and R2 is independently selected from the group comprising halogen, aryl-S-, heteroaryl-S-, aryl-NH-, heteroaryl-NH-, preferably thiazolyl-NH-, and -lower alkylene-R3, whereby R3 is selected from the group comprising heteroaryl, preferably imidazolyl and pyridyl, hydrogen and amino.

According to one aspect to the invention, R1 is selected from the group comprising hydrogen and hydroxy and R2 is -lower alkylene-R3, whereby R3 is amino.

According to one aspect to the invention, R1 is selected from the group comprising hydrogen and hydroxy and R2 is independently selected from the group comprising halogen, aryl, preferably phenylthio, amino, preferably thiazolylamino, and -lower alkylene-R3, whereby R3 is from the group comprising amino, hydrogen and heteroaryl, preferably imidazolyl.

According to one aspect to the invention, R1 is hydrogen or hydroxy and R2 is -lower alkylene-R3, whereby R3 is N-C3-C7 -cycloalkylamino, or N-C1-C4 alkyl-N-phenylthio-C1-C4 alkyl.

According to one aspect to the invention, R1 is hydroxy and R2 is -lower alkylene-R3, wherby R3 is amino, di-C1-C4 alkylamino, or N-C1-C4 alkyl-N-phenyl-C1-C4 alkylamino.

According to one aspect to the invention, R1 is hydroxy and R2 is -lower alkylene-R3, whereby R3 is imidazolyl that is bonded via a ring carbon or ring nitrogen atom and is unsubstituted or substituted by C1-C4 alkyl.

The present invention relates preferably to the use of a bisphosphonate derivative of Formula II (etidronate) 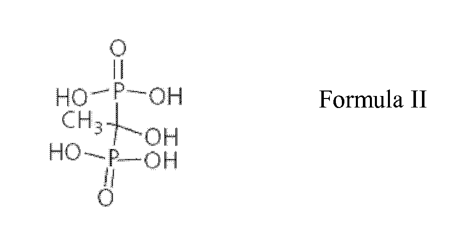 and/or a pharmaceutically acceptable salt thereof for use according to the present invention.

Furthermore, the present invention relates preferably to a composition comprising a bisphosphonate derivative of Formula II (etidronate) 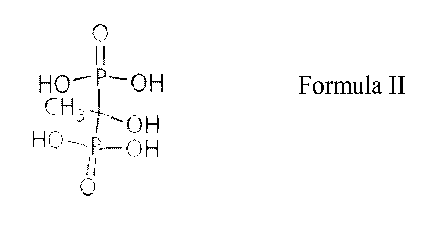 and/or a pharmaceutically acceptable salt thereof for a process according to the present invention.

The present invention relates preferably to the use of a bisphosphonate derivative of Formula III (clodronate) 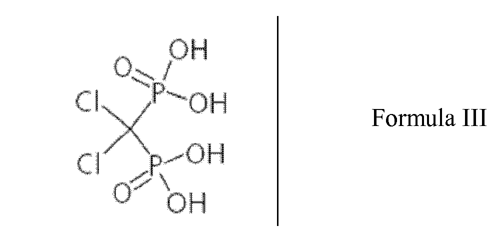 and/or a pharmaceutically acceptable salt thereof for use according to the present invention.

Furthermore, the present invention relates preferably to a composition comprising a bisphosphonate derivative of Formula III (clodronate) 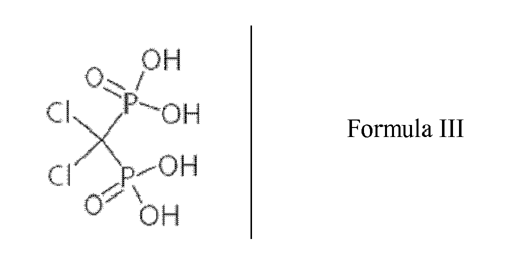 and/or a pharmaceutically acceptable salt thereof for a process according to the present invention.

The present invention relates preferably to the use of a bisphosphonate derivative of Formula IV (tiludronate) 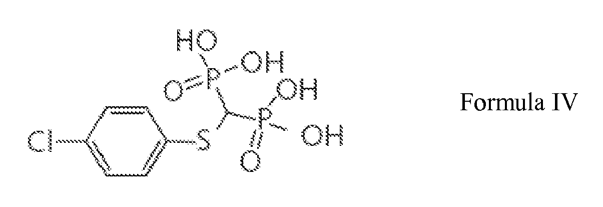 and/or a pharmaceutically acceptable salt thereof for use according to the present invention.

Furthermore, the present invention relates preferably to a composition comprising a bisphosphonate derivative of Formula IV (tiludronate) 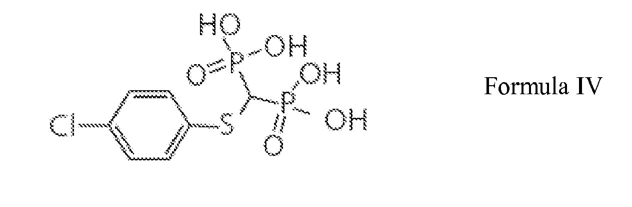 and/or a pharmaceutically acceptable salt thereof for a process according to the present invention.

The present invention relates preferably to the use of a bisphosphonate derivative of Formula V (pamidronate) 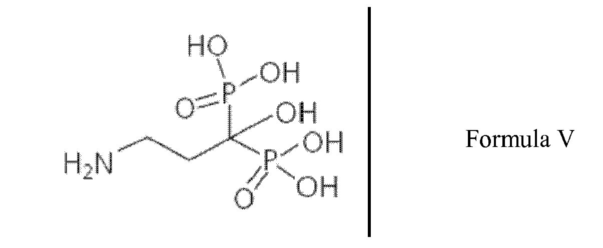 and/or a pharmaceutically acceptable salt thereof for use according to the present invention.

Furthermore, the present invention relates preferably to a composition comprising a bisphosphonate derivative of Formula V (pamidronate) 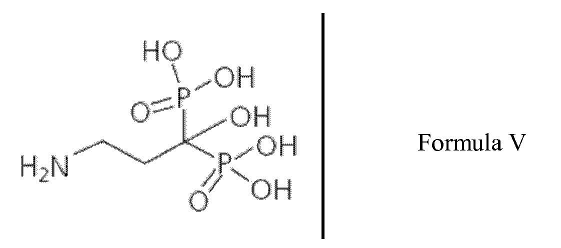 and/or a pharmaceutically acceptable salt thereof for a process according to the present invention.

The present invention relates preferably to the use of a bisphosphonate derivative of Formula VI (alendronate) 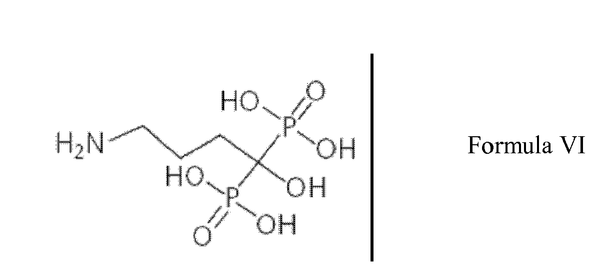 and/or a pharmaceutically acceptable salt thereof for use according to the present invention.

Furthermore, the present invention relates preferably to a composition comprising a bisphosphonate derivative of Formula VI (alendronate) 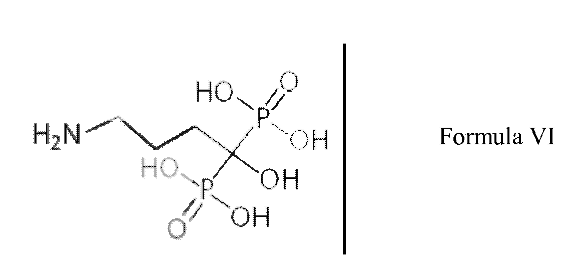 and/or a pharmaceutically acceptable salt thereof for a process according to the present invention.

The present invention relates preferably to the use of a bisphosphonate derivative of Formula VII (ibandronate) 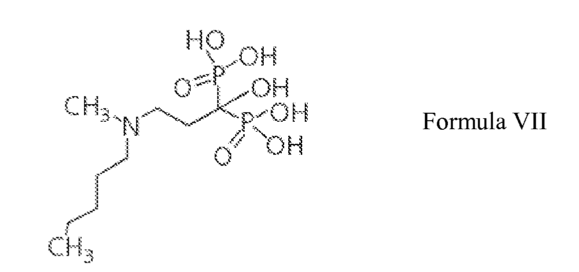 and/or a pharmaceutically acceptable salt thereof for use according to the present invention.

Furthermore, the present invention relates preferably to a composition comprising a bisphosphonate derivative of Formula VII (ibandronate) 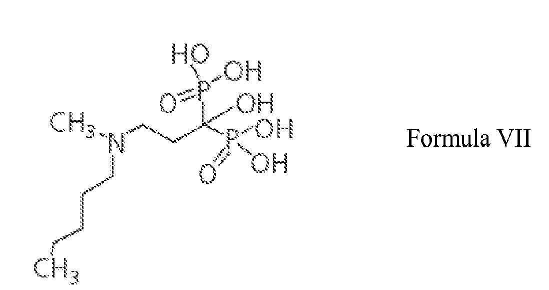 and/or a pharmaceutically acceptable salt thereof for a process according to the present invention.

The present invention relates preferably to the use of a bisphosphonate derivative of Formula VIII (risedronate) 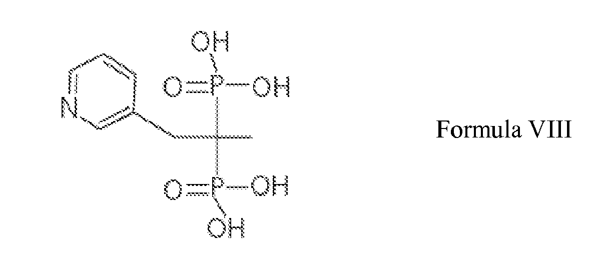 and/or a pharmaceutically acceptable salt thereof for use according to the present invention.

Furthermore, the present invention relates preferably to a composition comprising a bisphosphonate derivative of Formula VIII (risedronate) 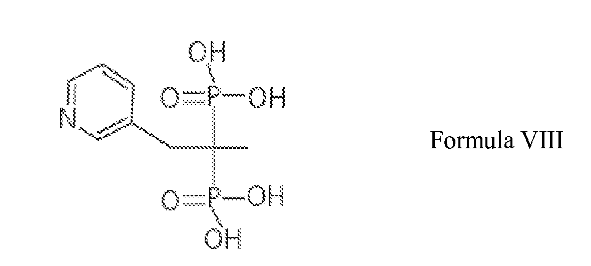 and/or a pharmaceutically acceptable salt thereof for a process according to the present invention.

The present invention relates preferably to the use of a bisphosphonate derivative of Formula IX (zoledronate) 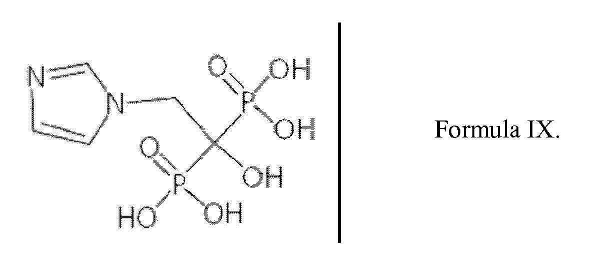 and/or a pharmaceutically acceptable salt thereof for use according to the present invention.

Furthermore, the present invention relates preferably to a composition comprising a bisphosphonate derivative of Formula IX (zoledronate) 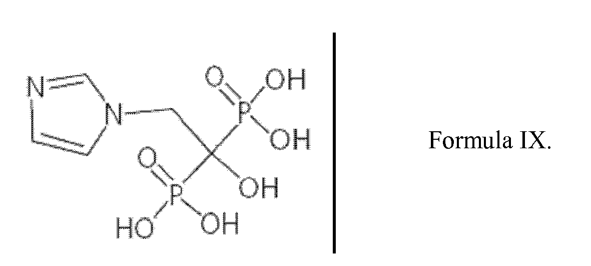 and/or a pharmaceutically acceptable salt thereof for a process according to the present invention.

The present invention relates preferably to the use of a bisphosphonate derivative of Formula X (neridronate) 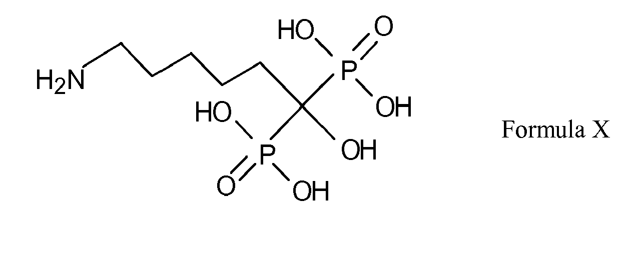 and/or a pharmaceutically acceptable salt thereof for use according to the present invention.

Furthermore, the present invention relates preferably to a composition comprising a bisphosphonate derivative of Formula X (neridronate) 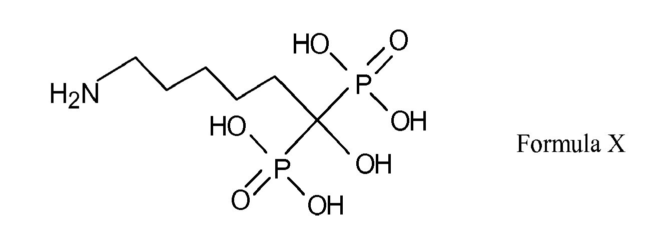 and/or a pharmaceutically acceptable salt thereof for a process according to the present invention.

The present invention relates preferably to the use of a bisphosphonate derivative of Formula XI (olpadronate) 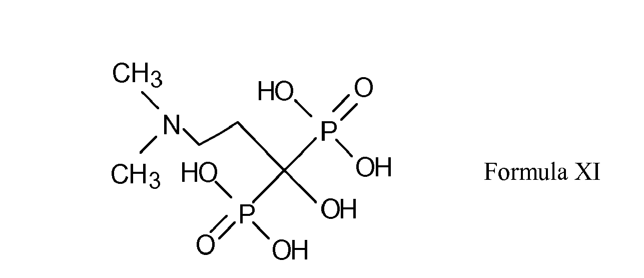 and/or a pharmaceutically acceptable salt thereof for use according to the present invention.

Furthermore, the present invention relates preferably to a composition comprising a bisphosphonate derivative of Formula XI (olpadronate) 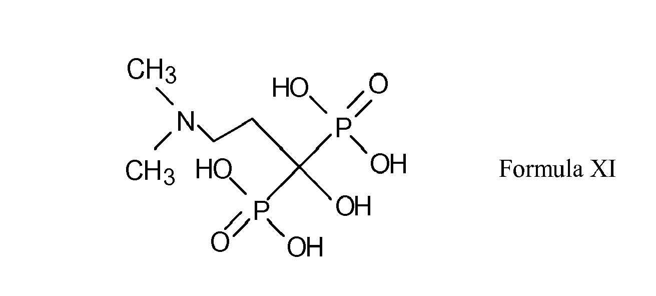 and/or a pharmaceutically acceptable salt thereof for a process according to the present invention.

Generic group definition: Throughout the description and claims generic groups have been used, for example alkyl, alkoxy, aryl. Unless otherwise specified the following are preferred groups that may be applied to generic groups found within compounds disclosed herein:
alkyl: linear and branched Cl-C8-alkyl,
alkenyl: C2-C6-alkenyl,
cycloalkyl: C3-C8-cycloalkyl,
alkoxy: Cl-C6-alkoxy,

alkylene: selected from the group consisting of:
methylene; 1,1-ethylene; 1,2-ethylene; 1,1-propylidene; 1,2-propylene; 1,3- propylene; 2,2-propylidene; butan-2-ol-1,4-diyl; propan-2-ol-1,3-diyl; 1, 4-butylene; cyclohexane-1,1-diyl; cyclohexan-1,2-diyl; cyclohexan-1,3- diyl; cyclohexan-1,4-diyl; cyclopentane-1,1-diyl; cyclopentan-1,2-diyl; and cyclopentan-1,3-diyl,
aryl: selected from homoaromatic compounds having a molecular weight under 300,
arylene: selected from the group consisting of: 1,2-phenylene; 1,3- phenylene; 1,4-phenylene; 1,2-naphtalenylene; 1,3-naphtalenylene; 1,4- naphtalenylene; 2,3-naphtalenylene; 1-hydroxy-2,3-phenylene; 1-hydroxy-2,4- phenylene; 1-hydroxy-2,5- phenylene; and 1-hydroxy-2,6-phenylene,
heteroaryl: selected from the group consisting of: unsubstituted or substituted, monocyclic, 5-or 6-membered monoaza-, diaza- or thiaza-aryl, pyridinyl; pyridyl; pyrimidinyl; pyrazinyl; triazolyl; pyridazinyl; 1,3,5-triazinyl; quinolinyl; isoquinolinyl; quinoxalinyl; imidazolyl; pyrazolyl; benzimidazolyl; thiazolyl; oxazolidinyl; pyrrolyl; carbazolyl; indolyl; and isoindolyl, wherein the heteroaryl may be connected to the compound via any atom in the ring of the selected heteroaryl,
heteroarylene: selected from the group consisting of: pyridindiyl; quinolindiyl; pyrazodiyl; pyrazoldiyl; triazolediyl; pyrazindiyl; and imidazolediyl, wherein the heteroarylene acts as a bridge in the compound via any atom in the ring of the selected heteroarylene, more specifically preferred are: pyridin-2, 3-diyl; pyridin-2,4-diyl; pyridin-2,5-diyl; pyridin-2,6-diyl; pyridin-3,4- diyl; pyridin-3,5-diyl; quinolin-2,3-diyl; quinolin-2,4-diyl; quinolin-2, 8-diyl; isoquinolin-1,3-diyl; isoquinolin-1,4-diyl; pyrazol-1,3-diyl; pyrazol-3,5- diyl; triazole-3,5-diyl; triazole-1,3-diyl; pyrazin-2,5-diyl; and imidazole-2,4-diyl, a -C1-C6-heterocycloalkyl, wherein the heterocycloalkyl of the -C1 -C6-heterocycloalkyl is, selected from the group consisting of: piperidinyl; piperidine; 1,4-piperazine, tetrahydrothiophene; tetrahydrofuran; 1,4,7-triazacyclononane; 1,4,8,11- tetraazacyclotetradecane; 1,4,7,10,13-pentaazacyclopentadecane; 1,4-diaza- 7-thia-cyclononane; 1,4- diaza-7-oxa-cyclononane; 1,4,7,10-tetraazacyclododecane; 1,4-dioxane; 1,4, 7-trithia-cyclononane; pyrrolidine; and tetrahydropyran, wherein the heterocycloalkyl may be connected to the -Cl-C6-alkyl via any atom in the ring of the selected heterocycloalkyl,
heterocycloalkylene: selected from the group consisting of: piperidin-1,2- ylene; piperidin-2,6-ylene; piperidin-4,4-ylidene; 1,4-piperazin-1,4-ylene; 1,4-piperazin-2,3-ylene; 1,4-piperazin-2,5-ylene; 1,4-piperazin-2,6-ylene; 1,4-piperazin- 1,2-ylene; 1,4-piperazin-1,3-ylene; 1,4-piperazin-1,4-ylene; tetrahydrothiophen-2,5-ylene; tetrahydrothiophen-3,4-ylene; tetrahydrothiophen-2,3-ylene; tetrahydrofuran-2,5-ylene; tetrahydrofuran- 3,4-ylene; tetrahydrofuran-2,3-ylene; pyrrolidin-2,5-ylene; pyrrolidin-3,4-ylene; pyrrolidin-2,3-ylene; pyrrolidin-1,2-ylene; pyrrolidin-1,3-ylene; pyrrolidin-2,2-ylidene; 1,4,7-triazacyclonon-1,4-ylene; 1,4,7- triazacyclonon-2,3-ylene; 1,4,7-triazacyclonon-2,9-ylene; 1,4,7-triazacyclonon-3,8-ylene; 1,4,7-triazacyclonon-2,2- ylidene; 1,4,8,11-tetraazacyclotetradec-1,4-ylene; 1,4,8,11- tetraazacyclotetradec-1,8-ylene; 1,4,8,11-tetraazacyclotetradec-2,3-ylene; 1,4,8,11-tetraazacyclotetradec-2,5-ylene; 1,4,8,11- tetraazacyclotetradec-1,2-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-1,2- ylene; 1,4,7,10-tetraazacyclododec-2,3- ylene; 1,4,7,10-tetraazacyclododec-2,2-ylidene; 1,4,7,10,13 pentaazacyclopentadec-1,4-ylene; 1,4,7,10,13- pentaazacyclopentadec-1,7-ylene; 1,4,7,10,13-pentaazacyclopentadec-2,3- ylene; 1,4,7,10,13-pentaazacyclopentadec-1,2-ylene; 1,4,7,10, 13-pentaazacyclopentadec-2,2-ylidene; 1,4-diaza-7-thia-cyclonon- 1,4-ylene; 1,4-diaza-7-thia-cyclonon-1,2-ylene; 1,4-diaza-7thia-cyclonon- 2,3-ylene; 1,4-diaza-7-thia-cyclonon-6,8-ylene; 1,4-diaza-7-thia-cyclonon- 2,2-ylidene; 1,4-diaza-7-oxacyclonon-1,4-ylene; 1,4-diaza-7-oxa-cyclonon- 1,2-ylene; l,4diaza-7-oxa-cyclonon-2,3-ylene; 1,4-diaza-7-oxa-cyclonon-6, 8-ylene; 1,4-diaza-7-oxa-cyclonon-2,2-ylidene; 1,4-dioxan-2,3-ylene; 1,4- dioxan-2,6-ylene; 1,4-dioxan-2,2-ylidene; tetrahydropyran-2,3-ylene; tetrahydropyran-2,6-ylene; tetrahydropyran-2,5-ylene; tetrahydropyran-2,2- ylidene; 1,4,7-trithia-cyclonon-2,3-ylene; 1,4,7-trithia-cyclonon-2,9- ylene; and 1,4,7-trithia-cyclonon-2,2-ylidene,
heterocycloalkyl: selected from the group consisting of: pyrrolinyl; pyrrolidinyl; morpholinyl; piperidinyl; piperazinyl; hexamethylene imine; 1,4-piperazinyl; tetrahydrothiophenyl; tetrahydrofuranyl; 1,4,7- triazacyclononanyl; 1,4,8,11-tetraazacyclotetradecanyl; 1,4,7,10,13-pentaazacyclopentadecanyl; 1,4-diaza-7-thiacyclononanyl; 1,4-diaza-7-oxa- cyclononanyl; 1,4,7,10-tetraazacyclododecanyl; 1,4-dioxanyl; 1,4,7- trithiacyclononanyl; tetrahydropyranyl; and oxazolidinyl, wherein the heterocycloalkyl may be connected to the compound via any atom in the ring of the selected heterocycloalkyl,
amine: the group -N(R)2 wherein each R is independently selected from: hydrogen; Cl-C6-alkyl; Cl-C6-alkyl-C6H5; and phenyl, wherein when both R are Cl-C6-alkyl both R together may form an - NC3 to an -NC5 heterocyclic ring with any remaining alkyl chain forming an alkyl substituent to the heterocyclic ring,
halogen: selected from the group consisting of: F; Cl; Br and I,
sulphonate: the group -S(O)2OR, wherein R is selected from: hydrogen; Cl- C6-alkyl; phenyl; Cl-C6-alkyl-C6H5; Li; Na; K; Cs; Mg; and Ca,
sulphate: the group -OS(O)2OR, wherein R is selected from: hydrogen; Cl- C6-alkyl; phenyl; Cl-C6-alkyl-C6H5; Li; Na; K; Cs; Mg; and Ca,
sulphone: the group -S(O)2R, wherein R is selected from: hydrogen; Cl-C6- alkyl; phenyl; Cl-C6-alkyl-C6H5 and amine (to give sulphonamide) selected from the group: -NR'2, wherein each R' is independently selected from: hydrogen; Cl-C6-alkyl; ClC6-alkyl-C6H5; and phenyl, wherein when both R' are Cl-C6-alkyl both R' together may form an -NC3 to an - NCS heterocyclic ring with any remaining alkyl chain forming an alkyl substituent to the heterocyclic ring,
carboxylate derivative: the group -C(O)OR, wherein R is selected from: hydrogen; Cl-C6-alkyl; phenyl; Cl-C6-alkyl-C6H5; Li; Na; K; Cs; Mg; and Ca,
carbonyl derivative: the group -C(O)R, wherein R is selected from: hydrogen; Cl-C6-alkyl; phenyl; Cl-C6-alkyl-C6H5 and amine (to give amide) selected from the group: -NR'2, wherein each R' is independently selected from: hydrogen; Cl- C6-alkyl; Cl-C6-alkyl-C6H5; and phenyl, wherein when both R' are Cl-C6- alkyl both R' together may form an -NC3 to an -NC5 heterocyclic ring with any remaining alkyl chain forming an alkyl substituent to the heterocyclic ring,
phosphonate: the group -P(O) (OR) 2, wherein each R is independently selected from: hydrogen; Cl-C6-alkyl; phenyl; Cl-C6-alkyl-C6H5; Li; Na; K; Cs; Mg; and Ca,
phosphate: the group -OP(O)(OR)2, wherein each R is independently selected from: hydrogen; Cl-C6-alkyl; phenyl; Cl-C6-alkyl-C6H5; Li; Na; K; Cs; Mg; and Ca,
phosphine: the group -P(R)2, wherein each R is independently selected from: hydrogen; Cl-C6-alkyl; phenyl; and Cl-C6-alkyl-C6H5,
phosphine oxide: the group -P (O) R2, wherein R is independently selected from: hydrogen; Cl-C6-alkyl; phenyl; and Cl-C6-alkyl-C6H5; and amine (to give phosphonamidate) selected from the group: -NR'2, wherein each R' is independently selected from: hydrogen; Cl-C6-alkyl; Cl-C6-alkyl-C6H5; and phenyl, wherein when both R' are Cl-C6-alkyl both R' together may form an -NC3 to an -NC5 heterocyclic ring with any remaining alkyl chain forming an alkyl substituent to the heterocyclic ring.

Unless otherwise specified the following are more preferred group restrictions that may be applied to groups found within compounds disclosed herein:
alkyl: linear and branched Cl-C6-alkyl,
alkenyl: C3-C6-alkenyl,
cycloalkyl: C6-C8-cycloalkyl,
alkoxy: Cl-C4-alkoxy,
aryl: selected from group consisting of: phenyl; biphenyl; phenylthio; naphthalenyl; anthracenyl; and phenanthrenyl,
arylene: selected from the group consisting of: 1,2-phenylene; 1,3- phenylene; 1,4-phenylene; 1,2-naphtalenylene; 1,4-naphtalenylene; 2,3- naphtalenylene and 1-hydroxy-2,6-phenylene,
heteroaryl: selected from the group consisting of:
   pyridinyl; pyrimidinyl; quinolinyl; pyrazolyl; triazolyl; isoquinolinyl; imidazolyl; and oxazolidinyl, unsubstituted or lower alkyl-substituted thiazolyl, wherein the heteroaryl may be connected to the compound via any atom in the ring of the selected heteroaryl, heteroarylene:
   selected from the group consisting of: pyridin 2,3-diyl; pyridin-2,4-diyl; pyridin-2,6-diyl; pyridin-3,5-diyl; quinolin-2,3-diyl; quinolin-2,4-diyl; isoquinolin-1,3-diyl; isoquinolin-1,4-diyl; pyrazol-3,5-diyl; and imidazole-2,4-diyl,

heterocycloalkyl: selected from the group consisting of:
pyrrolidinyl; morpholinyl; piperidinyl; piperidinyl; 1,4 piperazinyl; tetrahydrofuranyl; 1,4,7-triazacyclononanyl; 1,4,8,11-tetraazacyclotetradecanyl; 1,4,7,10,13-pentaazacyclopentadecanyl; 1,4,7,10-tetraazacyclododecanyl; and piperazinyl, wherein the heterocycloalkyl may be connected to the compound via any atom in the ring of the selected heterocycloalkyl, heterocycloalkylene: selected from the group consisting of:
   piperidin-2,6-ylene; piperidin-4,4-ylidene; 1,4-piperazin-1,4-ylene; 1,4-piperazin-2,3-ylene; 1,4-piperazin-2,6-ylene; tetrahydrothiophen-2,5-ylene; tetrahydrothiophen-3,4-ylene; tetrahydrofuran-2,5-ylene; tetrahydrofuran-3,4-ylene; pyrrolidin-2,5-ylene; pyrrolidin-2,2-ylidene; 1,4,7-triazacyclonon-1,4- ylene; 1,4,7-triazacyclonon-2,3-ylene; 1,4,7-triazacyclonon-2,2-ylidene; 1,4,8,11- tetraazacyclotetradec-1,4-ylene; 1,4,8,11-tetraazacyclotetradec-1,8-ylene; 1,4,8,11-tetraazacyclotetradec-2,3-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-2,3-ylene; 1,4,7,10-tetraazacyclododec-2,2-ylidene; 1,4,7,10,13- pentaazacyclopentadec-1,4-ylene; 1,4,7,10,13-pentaazacyclopentadec-1,7-ylene; 1,4-diaza-7-thia-cyclonon-1,4 ylene; 1,4-diaza-7-thiacyclonon-2,3-ylene; 1,4-diaza-7-thia cyclonon-2,2-ylidene; 1,4-diaza-7-oxa-cyclonon-1,4-ylene; 1,4 diaza-7-oxa-cyclonon-2,3-ylene;l,4-diaza-7-oxa-cyclonon-2,2- ylidene; 1,4-dioxan-2,6-ylene; 1,4-dioxan-2,2-ylidene; tetrahydropyran-2,6-ylene; tetrahydropyran-2,5-ylene; and tetrahydropyran- 2,2-ylidene, a -Cl-C6-alkyl-heterocycloalky, wherein the heterocycloalkyl of the -Cl- C6-heterocycloalkyl is selected from the group consisting of:
   piperidinyl; 1,4-piperazinyl; tetrahydrofuranyl; 1,4,7- triazacyclononanyl; 1,4,8,11-tetraazacyclotetradecanyl; 1,4,7,10,13- pentaazacyclopentadecanyl; 1,4,7,10-tetraazacyclododecanyl; and pyrrolidinyl, wherein the heterocycloalkyl may be connected to the -Cl-C6- alkyl via any atom in the ring of the selected heterocycloalkyl,
amine: the group -N (R) 2, wherein each R is independently selected from: hydrogen; Cl-C6-alkyl; and benzyl,
halogen: selected from the group consisting of: F and Cl,
sulphonate: the group -S(O)2OR, wherein R is selected from: hydrogen; Cl- C6-alkyl; Na; K; Mg; and Ca,
sulphate: the group -OS(O)2OR, wherein R is selected from: hydrogen; Cl- C6-alkyl; Na; K; Mg; and Ca,
sulphone: the group -S(O)2R, wherein R is selected from: hydrogen; Cl-C6- alkyl; benzyl and amine selected from the group: -NR'2, wherein each R' is independently selected from: hydrogen; Cl-C6-alkyl; and benzyl,
carboxylate derivative: the group -C(O)OR, wherein R is selected from hydrogen; Na; K; Mg; Ca; Cl-C6-alkyl; and benzyl,
carbonyl derivative: the group: -C(O)R, wherein R is selected from: hydrogen; Cl-C6-alkyl; benzyl and amine selected from the group: -NR'2, wherein each R' is independently selected from: hydrogen; Cl-C6-alkyl; and benzyl,
phosphonate: the group -P(O) (OR)2, wherein each R is independently selected from: hydrogen; Cl-C6-alkyl; benzyl; Na; K; Mg; and Ca,
phosphate: the group -OP(O) (OR)2, wherein each R is independently selected from: hydrogen; Cl-C6-alkyl; benzyl; Na; K; Mg; and Ca,
phosphine: the group -P(R)2, wherein each R is independently selected from: hydrogen; Cl-C6-alkyl; and benzyl,
phosphine oxide: the group -P(O)R2, wherein R is independently selected from: hydrogen; Cl-C6-alkyl; benzyl and amine selected from the group: -NR'2, wherein each R' is independently selected from: hydrogen; Cl-C6-alkyl; and benzyl.
M, Mₙ (n being an integer): Metals (either charged or uncharged), whereby two Metals Mₙ and Mₘ are independently selected from each other unless otherwise indicated.

Salts of bisphosphonate derivatives of Formula I are especially their salts with bases, such as metal salts derived from groups Ia, Ib, IIa and IIb of the Periodic Table of Elements, for example alkali metal salts, especially sodium or potassium salts, alkaline earth metal salts, especially calcium or magnesium salts, and also ammonium salts with ammonia or organic amines.

Radicals or compounds designated "lower" contain especially up to and include 7 carbon atoms:

Lower alkyl is, for example, methyl, ethyl, n-propyl, isopropyl, n-butyl, isobutyl, sec.-and tert.-butyl, and also includes corresponding pentyl, hexyl and heptyl radicals. C1-C4 alkyl is preferred.

Lower alkoxy is, for example, methoxy, ethoxy, n-propoxy, isopropoxy and n-butoxy, and also includes corresponding pentyloxy, hexyloxy and heptyloxy radicals. C1-C4 alkoxy is preferred.

Lower alkylene is straight-chain or branched and is, for example, C1-C7 alkylene, such as methylene, ethylene, propylene, butylene, pentylene and hexylene and heptylene, and also 2-methyl-1,3-propylene and 2,4- or 1,5-dimethyl-1,5-pentylene. Lower alkylene as a substituent of disubstituted amino R3 contains at least two carbon atoms, preferably from 4 to 6 carbon atoms. In amino that is disubstituted by lower alkylene, lower alkylene linking two lower alkylene carbon atoms contains especially up to and including 5 carbon atoms and is preferably methylene.

Depending on the starting materials and procedures chosen, the compounds of general Formula I may be in the form of one of the possible isomers or in the form of a mixture thereof, for example in the form of optical isomers, such as enantiomers or diastereoisomers, or geometrical isomers, such as cis-trans isomers. The optical isomers are in the form of the pure antipodes and/or of racemates.

The compounds of general Formula I may also be employed in the form of their hydrates or include other solvents used for crystallisation.

The invention relates specifically to the pharmaceuticals described in the examples.

Surprisingly, the inventors of the present invention have found recoloration effects by physiological repigmentation of "grayed" hair due to a treatment with a bisphosphonate derivative according to the present invention.

The solution according to the present invention has at least one of the following advantages:

A particular advantage of a bisphosphonate derivative mentioned above and/or a pharmaceutically acceptable salt thereof for use according to the present invention is that the recoloration effect for "grayed" hair due to physiological repigmentation is permanent.

Another particular advantage of a bisphosphonate derivative mentioned above and/or a pharmaceutically acceptable salt thereof for use according to the present invention is the reachievement of the visual effect of the natural hair color.

Another particular advantage of a bisphosphonate derivative mentioned above and/or a pharmaceutically acceptable salt thereof for use according to the present invention is the maintenance of the hair quality.

In one aspect of the present invention the administration of a pharmaceutical containing a bisphosphonate derivative of general Formula I and/or a pharmaceutically acceptable salt thereof for use according to the invention is an enteral, a parenteral and/or a topical administration.

Furthermore, the present invention relates to a process according to the present invention, wherein the administration of the pharmaceutical is an enteral, a parenteral and/or a topical administration.

The (topically) administrable pharmaceuticals according to the invention contain a bisphosphonate derivative of general Formula I, for example, in a pharmacologically effective amount, together with a pharmaceutically acceptable additive or adjunct. In the following the term "active ingredient" should mean a bisphosphonate derivative of general Formula I and/or a pharmaceutically acceptable salt thereof. The daily dose of active ingredient depends on age and individual condition and on the method of administration.

In one aspect of the present invention pharmaceuticals suitable for topical administration for use according to the invention are especially creams, ointments and gels and also pastes, foams, tinctures and solutions that contain from approximately 0.5 to approximately 5% active ingredient.

Furthermore, the present invention relates to a process according to the present invention, wherein the pharmaceutical is a cream, an ointment, a gel, a paste, a foam, a tincture and/or a solution that contain(s) from approximately 0.5 to approximately 5% active ingredient for a topical administration.

Creams or lotions are oil-in-water emulsions that contain more than 50% water. As oily base there are used especially fatty alcohols, especially those containing from 12 to 18 carbon atoms, for example lauryl, cetyl or stearyl alcohol, fatty acids, especially those containing from 10 to 18 carbon atoms, for example palmitic or stearic acid, liquid to solid waxes, for example isopropyl myristate, wool wax or beeswax, and/or hydrocarbons, especially liquid, semi-solid or solid substances or mixtures thereof, for example petroleum jelly (petrolatum) or paraffin oil. Suitable emulsifiers are surface-active substances having predominantly hydrophilic properties, such as corresponding non-ionic emulsifiers, for example fatty acid esters of polyalcohols or ethylene oxide adducts thereof, especially corresponding fatty acid esters with (poly)ethylene glycol, (poly)propylene glycol or sorbitol, the fatty acid moiety containing especially from 10 to 18 carbon atoms, especially partial glycerol fatty acid esters or partial fatty acid esters of polyhydroxyethylene sorbitan, such as polyglycerol fatty acid esters or polyoxyethylene sorbitan fatty acid esters (Tweens), and also polyoxyethylene fatty alcohol ethers or fatty acid esters, the fatty alcohol moiety containing especially from 12 to 18 carbon atoms and the fatty acid moiety especially from 10 to 18 carbon atoms, especially those having approximately from 2 to 23 ethylene glycol or ethylene oxide units, such as polyhydroxyethylenecetylstearyl ether (for example Cetomacrogol), polyhydroxyethylene-(4)-lauryl ether and polyhydroxyethyleneglycerol fatty acid ester (for example Tagat S), or corresponding ionic emulsifiers, such as alkali metal salts of fatty alcohol sulfates, especially having from 12 to 18 carbon atoms in the fatty alcohol moiety, for example sodium lauryl sulfate, sodium cetyl sulfate or sodium stearyl sulfate, which are usually used in the presence of fatty alcohols, for example cetyl alcohol or stearyl alcohol. Additives to the aqueous phase are, inter alia, agents that prevent the creams from drying out, for example humectants, such as polyalcohols, such as glycerol, sorbitol, propylene glycol and/or polyethylene glycols, and also preservatives, perfumes, etc.

Ointments or lotions are water-in-oil emulsions that contain up to 70%, but preferably from approximately 20% to approximately 50%, water or aqueous phase. Suitable as fatty phase are especially hydrocarbons, for example petroleum jelly, paraffin oil and/or hard paraffins, which, in order to improve the water-binding capacity, preferably contain suitable hydroxy compounds, such as fatty alcohols or esters thereof, for example cetyl alcohol or wool wax alcohols, or wool wax. Emulsifiers are corresponding lipophilic substances, for example of the type indicated above, such as sorbitan fatty acid esters (Spans), for example sorbitan oleate and/or sorbitan isostearate. Additives to the aqueous phase are, inter alia, humectants, such as polyalcohols, for example glycerol, propylene glycol, sorbitol and/or polyethylene glycol, and also preservatives, perfumes, etc.

Microemulsions are isotropic systems based on the following four components: water, an emulsifier, for example of the type indicated above, such as a surfactant, for example emulgin, a lipid, such as a non-polar oil, for example paraffin oil, and an alcohol containing a lipophilic group, for example 2-octyldodecanol. If desired, other additives may be added to the microemulsions.

Fatty ointments are water-free and contain as base especially hydrocarbons, for example paraffin, petroleum jelly and/or liquid paraffins, also natural or partially synthetic fat, such as fatty acid esters of glycerol, for example coconut fatty acid triglyceride, or preferably hardened oils, for example hydrogenated groundnut oil or castor oil, also fatty acid partial esters of glycerol, for example glycerol mono- and di-stearate, and also, for example, the fatty alcohols increasing the water-absorption capacity, emulsifiers and/or additives mentioned in connection with the ointments.

With gels, a distinction is made between aqueous gels, water-free gels and gels having a low water content, which gels consist of swellable, gel-forming materials. There are used especially transparent hydrogels based on inorganic or organic macromolecules. High molecular weight inorganic components having gel-forming properties are predominantly water-containing silicates, such as aluminium silicates, for example bentonite, magnesium aluminium silicates, for example Veegum, or colloidal silicic acid, for example Aerosil. As high molecular weight organic substances there are used, for example, natural, semi-synthetic or synthetic macromolecules. Natural and semi-synthetic polymers are derived, for example, from polysaccharides containing a great variety of carbohydrate components, such as celluloses, starches, tragacanth, gum arabic and agar-agar, and gelatin, alginic acid and salts thereof, for example sodium alginate, and derivatives thereof, such as lower alkylcelluloses, for example methyl- or ethyl-cellulose, carboxy- or hydroxy-lower alkylcelluloses, for example carboxymethyl- or hydroxyethyl-cellulose. The components of synthetic gel-forming macromolecules are, for example, suitably substituted unsaturated aliphatic compounds such as vinyl alcohol, vinylpyrrolidine, acrylic or methacrylic acid. Examples of such polymers are polyvinyl alcohol derivatives, such as polyviol, polyvinylpyrrolidines, such as collidone, polyacrylates and polymethacrylates, especially having a molecular weight of from approximately 80000 to approximately 1 million, or salts thereof, such as Rohagit S, Eudispert or Carbopol. Customary additives, such as preservatives or perfumes, may be added to the gels.

Foams are administered, for example, from pressurised containers and are liquid oil-in-water emulsions in aerosol form; unsubstituted or halogenated hydrocarbons, such as alkanes, for example propane or butane, or chlorofluoro-lower alkanes, for example dichlorodifluoromethane and dichlorotetrafluoroethane, are used as propellant. As oil phase there are used, inter alia, hydrocarbons, for example paraffin oil, fatty alcohols, for example cetyl alcohol, fatty acid esters, for example isopropyl myristate, and/or other waxes. As emulsifiers there are used, inter alia, mixtures of emulsifiers having predominantly hydrophilic properties, such as polyoxyethylene sorbitan fatty acid esters (Tweens), and emulsifiers having predominantly lipophilic properties, such as sorbitan fatty acid esters (Spans). The customary additives, such as preservatives, etc., are also added.

Tinctures and solution generally have an ethanolic base, to which water may be added and to which there are added, inter alia, polyalcohols, for example glycerol, glycols and/or polyethylene glycol, as humectants for reducing evaporation, and fat-restoring substances, such as fatty acid esters with low molecular weight polyethylene glycols, that is to say lipophilic substances that are soluble in the aqueous mixture, as a replacement for the fatty substances removed from the skin by the ethanol, and, if necessary, other adjuncts and additives. Suitable tinctures or solutions may also be applied in spray form by means of suitable devices.

The manufacture of the topically administrable pharmaceuticals is effected in a manner known per se, for example by dissolving or suspending the active ingredient in the base or, if necessary, in a portion thereof. When the active ingredient is administered in the form of a solution, it is generally dissolved in one of the two phases before emulsification; when the active ingredient is administered in the form of a suspension, it is mixed with a portion of the base after emulsification and then added to the remainder of the formulation.

Suitable penetration enhancers are preferably monovalent, saturated or unsaturated aliphatic, cycloaliphatic or aromatic alcohols having from 4 to 12 carbon atoms, e.g. n-hexanol or cyclohexanol, aliphatic, cycloaliphatic or aromatic hydrocarbons having from 5 to 12 carbon atoms, e.g. hexane, cyclohexane, isopropylbenzene and the like, cycloaliphatic or aromatic aldehydes and ketones having from 4 to 10 carbon atoms, such as cyclohexanone, acetamide, N,N-di-lower alkylacetamides such as N,N-dimethylacetamide or N,N-diethylacetamide, C10 -C20 alkanoylamides, e.g. N,N-dimethyllauroylamide, 1-n-C10 -C20 alkylazacycloheptan-2-one, e.g. 1-n-dodecylazacycloheptan-2-one (Azone.RTM., Nelson), pyrrolidones, such as N-methylpyrrolidone, polyalkylene glycol laureates, e.g. polyethylene glycol monolaureate or N-2-hydroxyethylacetamide, and known vehicles and/or penetration enhancers such as aliphatic, cycloaliphatic and aromatic esters, N,N-di-lower alkyl sulfoxide, unsaturated oils, halogenated or nitrated aliphatic or cycloaliphatic hydrocarbons, salicylates, polyalkylene glycol silicates, and mixtures thereof.

C2-C4 alkanols, e.g. isopropanol or isobutanol and, especially, ethanol, are especially preferred as penetration enhancers.

Adjuncts can be added to the active ingredients for enteral, a parenteral and/or a topical administration. Suitable adjuncts are water, isotonic aqueous sodium chloride solution, dextrose in water or sodium chloride solution, liquid glyceryl triesters with low molecular weight fatty acids, lower alkanols, natural oils such as corn oil, groundnut oil, sesame oil, castor oil and condensation products thereof with ethylene oxide, and the like, hydrocarbons such as pharmaceutical grade mineral oil, silicones, emulsifiers such as monoglycerides or diglycerides of fatty acids, phospholipic acid derivatives such as lecithin or cephalin, polyalkylene glycols such as polyethylene glycol, aqueous phases to which a swelling agent such as sodium carboxymethylcellulose, sodium alginate, polyvinylpolypyrrolidone, etc. has been added and to which, in addition, dispersion agents or emulsifiers such as lecithin may be added, polyoxyethylene and the like. The adjuncts may, in addition, contain additives such as preservatives, stabilisers, wetting agents, emulsifiers, etc.

If C2-C4 alkanols such as ethanol are used as penetration enhancers, gelling agents such as gelatin or swelling agents such as cellulose ethers, e.g. hydroxypropylcellulose, are preferably added as adjuncts to the active ingredient formulation.

The daily dose of active ingredient for a patient weighing about 70 kg is estimated to be from approximately 50 µg to approximately 150 µg, depending on the potency of the particular active ingredient used.

The invention relates as well to the use of a bisphosphonate derivative according to general Formula I and/or a pharmaceutically acceptable salt thereof for a topical administration.

Furthermore, the present invention relates to a process, comprising: administering topically a a bisphosphonate derivative according to general Formula I and/or a pharmaceutically acceptable salt thereof to a human.

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, features, characteristics and advantages of the object of the invention are disclosed in the subclaims and the following description of examples, which --in an exemplary fashion-- show several embodiments and examples of a claimed bisphosphonate derivative for use according to the invention.

### Examples: Case study

The invention is based on a case-observation in a myeloma patient (male, * 10.11.1941) on monotherapy with zoledronate. Multiple myeloma is a non-Hodgkin lymphoma and represents one of the most frequent malignant bone diseases.

Multiple myeloma in Stage I according to Durie and Salmon without osteolytic activity was diagnosed in the patient in November 2006. Since Stage I myeloma without clinically relevant symptoms are generally not aggressively treated with cytotostatic substances, the patient was included in an experimental monotherapy scheme applying Zometa® (active ingriedient: zoledronate), 4 mg i.v. in calcium-free infusion solution on a once monthly basis.

Zoledronate treatment is pursued for 6 months with a total of 6 therapy cycles until progression of myeloma (proliferation of plasma cell population in the bone marrow) made a more aggressive therapy approach necessary.

A suprising side-effect was observed:

The patient who was completely white-haired at the time of therapy induction, increasingly developed black scalp hair which, after 3-4 months of zoledronate treatment, switched the former complete white appearance to a natural-looking dark-greyish shape. The patient reported that he was black-haired during his teens and twens.

Since zoledronate was applied in monotherapy and no other relevant measures were deployed, the surprising change in hair coloration in the respective patient was caused by the bisphosphonate therapy of the underlying myeloma disease.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto.

### REFERENCES

1. Agrup G, Hansson C, Rorsman H, Rosengren AM, Rosengren E. 1978 Trichochromes in red hair. Acta Derm Venereol. 58(4):357-8.
2. Fleisch H. 2002 The role of bisphosphonates in breast cancer: Development of bisphosphonates. Breast Cancer Res. 4(1):30-34.
3. Jeal W, Barradell LB, McTavish D. 1997 Alendronate. A review of its pharmacological properties and therapeutic efficacy in postmenopausal osteoporosis. Drugs. 53(3):415-34.
4. Peter CP, Handt LK, Smith SM. 1998 Esophageal irritation due to alendronate sodium tablets: possible mechanisms. Dig Dis Sci. 43(9):1998-2002.
5. Peter CP, Kindt MV, Majka JA. 1998 Comparative study of potential for bisphosphonates to damage gastric mucosa of rats. Dig Dis Sci. 43(5):1009-15.
6. Rosen CJ, Holick MF, Millard PS. 1994 Premature graying of Hair Is a Risk Marker for Osteopenia. J Clin Endocrinol Metab. 79(3):854-857.
7. Slominski A, Wortsman J, Plonka PM, Schallreuter KU, Plaus R, Tobin DJ. 2005 Hair follicle pigmentation. J Invest Dermatol. 124(1):12-21.
8. Tobin DJ, Paus R. 2001 Graying: gerontobiology of the hair follicle pigmentary unit. Exp Gerontol. 36(1):29-54.
9. Trüeb RM. 2005 Aging of hair. J Cosmet Dermatol. 4(2):60-72.
10. EP 1 008 592 A2 (Fujirebio Kabushiki Kaisha) 14 June 2000
11. WO 97/44017 (MERCK & CO., INC.) 27 November 1997
12. WO 2007/031785 (SELAMINE LTD) 22 March 2007

## Claims

1. Use of a bisphosphonate derivative according to general Formula I 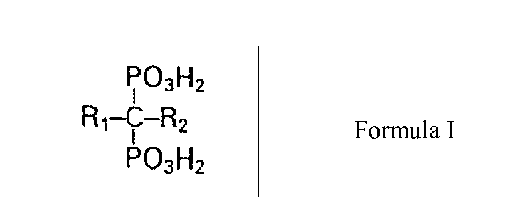 and/or a pharmaceutically acceptable salt thereof,
wherein R₁ and/or R₂ are independently selected out of a group comprising hydrogen, hydroxyl, halogen, pseudohalogen, formyl, carboxy- and/or carbonyl derivatives, alkyl, long-chain alkyl, alkoxy, long-chain alkoxy, cycloalkyl, halogenalkyl, aryl, arylene, halogenaryl, heteroaryl, heteroarylene, heterocycloalkylene, heterocycloalkyl, halogenheteroaryl, alkenyl, halogenalkenyl, alkinyl, halogenalkinyl, keto, ketoaryl, halogenketoaryl, ketoheteroaryl, ketoalkyl, halogenketoalkyl, ketoalkenyl, halogenketoalkenyl, phosphoalkyl, phosphonate, phosphate, phosphine, phosphine oxide, phosphoryl, phosphoaryl, sulphonyl, sulphoalkyl, sulphoarenyl, sulphonate, sulphate, sulphone, amine, polyether, silylalkyl, silylalkyloxy, and wherein at appropriate residues one or more non-adjacent CH₂-groups may be replaced independently from each other with -O-, -S-, -NH-, -NR°-, -SiR°R°°-, -CO-, -COO-, -OCO-, -OCO-O-, -SO₂-, -S-CO-, -CO-S-, -CY¹=CY² or -C=C- in that way that O and/or S-atoms are not connected directly with each other, likewise replaced optionally with aryl- or heteroaryl which preferably contain 1 to 30 C-atoms (terminal CH₃-groups are meant as CH₂-groups in terms of CH₂-H), wherein R°, R°°, Y¹ and Y² are independently selected out of a group comprising alkyl and aryl,
in the manufacture of a pharmaceutical for the treatment of age-related and/or premature graying of hair.

2. Use of a bisphosphonate derivative according to claim 1, wherein R1 is selected from the group comprising halogen, hydrogen and hydroxy and R2 is independently selected from the group comprising halogen, aryl-S-, heteroaryl-S-, aryl-NH-, heteroaryl-NH-, preferably monocyclic, 5- or 6-membered monoaza-, diaza- or thiaza-aryl-NH-, and -alkylene-R3, whereby R3 is selected from the group comprising heteroaryl, hydrogen and amino.

3. Use of a bisphosphonate derivative according to any of the preceding claims, wherein R1 is selected from the group comprising halogen, hydrogen and hydroxy and R2 is independently selected from the group comprising halogen, aryl-S-, heteroaryl-S-, aryl-NH-, heteroaryl-NH-, preferably thiazolyl-NH-, and -lower alkylene-R3, whereby R3 is selected from the group comprising heteroaryl, preferably imidazolyl and pyridyl, hydrogen and amino.

4. Use of a bisphosphonate derivative according to any of the preceding claims, wherein R1 is selected from the group comprising hydrogen and hydroxy and R2 is -lower alkylene-R3, whereby R3 is amino.

5. Use of a bisphosphonate derivative according to any of the preceding claims, wherein R1 is selected from the group comprising hydrogen and hydroxy and R2 is independently selected from the group comprising halogen, aryl, preferably phenylthio, amino, preferably thiazolylamino, and -lower alkylene-R3, whereby R3 is from the group comprising amino, hydrogen and heteroaryl, preferably imidazolyl.

6. Use of a bisphosphonate derivative according to any of the preceding claims, wherein R1 is hydrogen or hydroxy and R2 is -lower alkylene-R3, whereby R3 is N-C3-C7 - cycloalkylamino, or N-C1-C4 alkyl-N-phenylthio-C1-C4 alkyl.

7. Use of a bisphosphonate derivative according to any of the preceding claims, wherein R1 is hydroxy and R2 is -lower alkylene-R3, wherby R3 is amino, di-C1-C4 alkylamino, or N-C1-C4 alkyl-N-phenyl-C1-C4 alkylamino.

8. Use of a bisphosphonate derivative according to any of the preceding claims, wherein R1 is hydroxy and R2 is -lower alkylene-R3, whereby R3 is imidazolyl that is bonded via a ring carbon or ring nitrogen atom and is unsubstituted or substituted by C 1-C4 alkyl.

9. Use of a bisphosphonate derivative according to any of the preceding claims, wherein the bisphosphonate derivative has the Formula:
a) (etidronate) 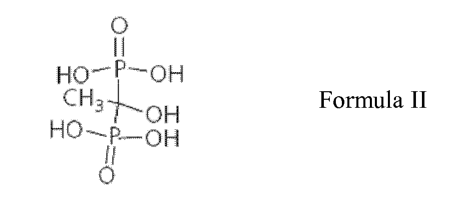
b) (clodronate) 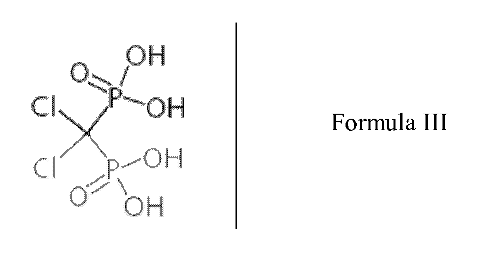
c) (tiludronate) 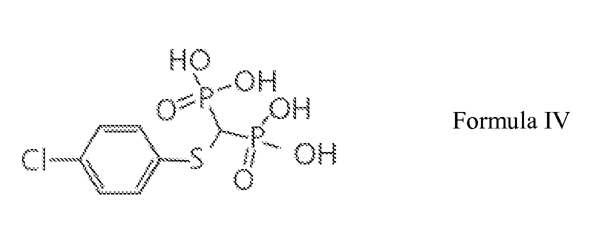
d) (pamidronate) 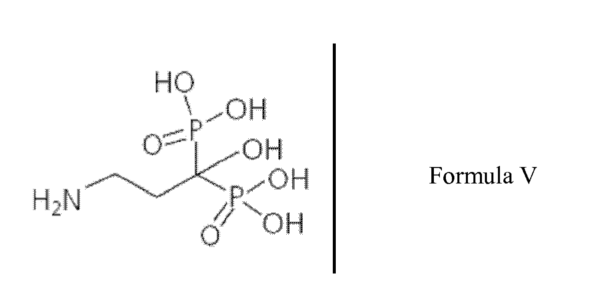
e) (alendronate) 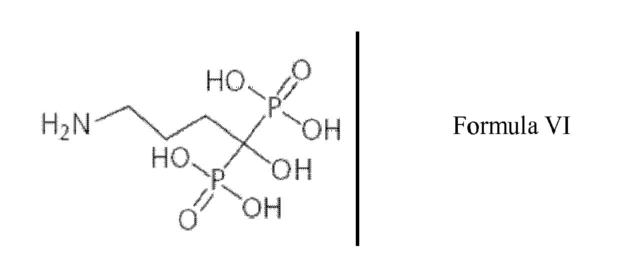
f) (ibandronate) 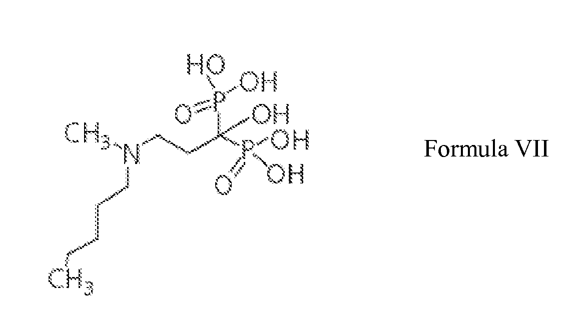
g) (risedronate) 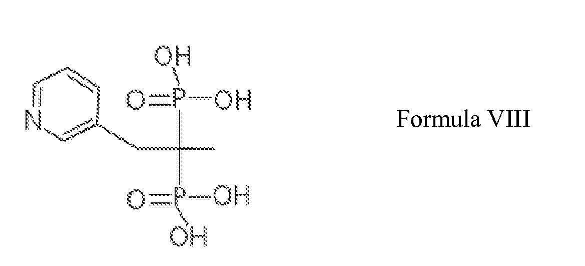
h) (zoledronate) 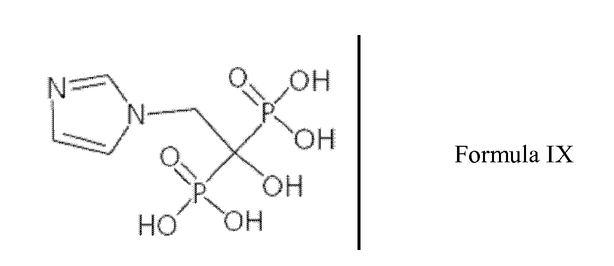
i) (neridronate) 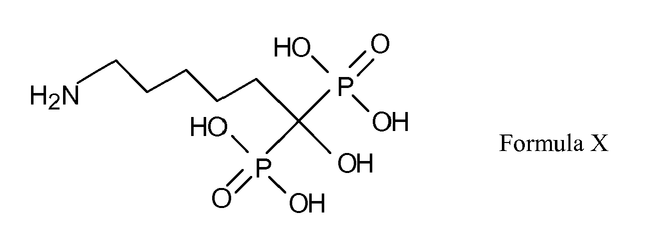 and/or
j) (olpadronate)

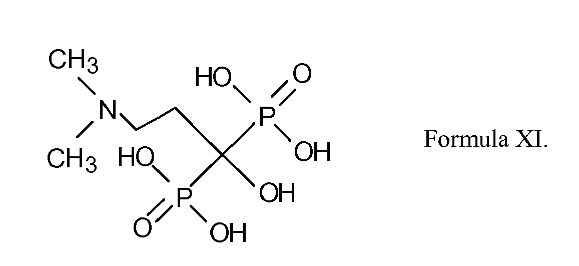

10. Use of a bisphosphonate derivative according to any of the preceding claims, wherein the administration of the pharmaceutical is an enteral, a parenteral and/or a topical administration.

11. Use of a bisphosphonate derivative according to claim 10, wherein the pharmaceutical is a cream, an ointment, a gel, a paste, a foam, a tincture and/or a solution that contain(s) from approximately 0.5 to approximately 5% active ingredient for a topical administration.

12. Use of a bisphosphonate derivative according to general Formula I 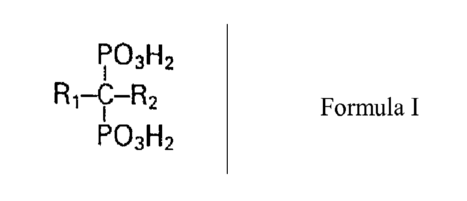 and/or a pharmaceutically acceptable salt thereof,
wherein R₁ and/or R₂ are independently selected out of a group comprising hydrogen, hydroxyl, halogen, pseudohalogen, formyl, carboxy- and/or carbonyl derivatives, alkyl, long-chain alkyl, alkoxy, long-chain alkoxy, cycloalkyl, halogenalkyl, aryl, arylene, halogenaryl, heteroaryl, heteroarylene, heterocycloalkylene, heterocycloalkyl, halogenheteroaryl, alkenyl, halogenalkenyl, alkinyl, halogenalkinyl, keto, ketoaryl, halogenketoaryl, ketoheteroaryl, ketoalkyl, halogenketoalkyl, ketoalkenyl, halogenketoalkenyl, phosphoalkyl, phosphonate, phosphate, phosphine, phosphine oxide, phosphoryl, phosphoaryl, sulphonyl, sulphoalkyl, sulphoarenyl, sulphonate, sulphate, sulphone, amine, polyether, silylalkyl, silylalkyloxy, and wherein at appropriate residues one or more non-adjacent CH₂-groups may be replaced independently from each other with -O-, -S-, -NH-, -NR°-, -SiR°R°°-, -CO-, -COO-, -OCO-, -OCO-O-, -SO₂-, -S-CO-, -CO-S-, -CY¹=CY² or -C=C- in that way that O and/or S-atoms are not connected directly with each other, likewise replaced optionally with aryl- or heteroaryl which preferably contain 1 to 30 C-atoms (terminal CH₃-groups are meant as CH₂-groups in terms of CH₂-H), wherein R°, R°°, Y¹ and Y² are independently selected out of a group comprising alkyl and aryl,
for a topical administration.
